# EUROPEAN PATENT SPECIFICATION

(11) **EP 1 154 021 B1**
(45) Date of publication and mention of the grant of the patent: **26.05.2010**
(21) Application number: 01108168.4
(22) Date of filing: 30.03.2001
(51) Int. Cl.: C12N 15/54, C12N 5/10, C12N 9/10, C12P 7/62

(54) **Polyhydroxyalkanoate synthase and gene encoding the same enzyme**
Polyhydroxyalkanoat-Synthase und dieses Enzym kodierendes Gen
Polyhydroxyalcanoate synthase et gène codant pour cette enzyme

(30) Priority: 30.03.2000 JP 2000095003
(43) Date of publication of application: 14.11.2001
(73) Proprietor: CANON KABUSHIKI KAISHA, Tokyo (JP)
(72) Inventor: Yano, Tetsuya, Ohta-ku, Tokyo (JP); Imamura, Takeshi, Ohta-ku, Tokyo (JP); Suda, Sakae, Ohta-ku, Tokyo (JP); Honma, Tsutomu, Ohta-ku, Tokyo (JP)
(74) Representative: Weser, Wolfgang

(56) References cited:
- EP-A- 0 897 005
- WO-A-01/11014
- WO-A-91/00917
- US-A- 5 849 894
- MATSUSAKI HIROMI ET AL: "Cloning and molecular analysis of the poly(3-hydroxybutyrate) and poly(3-hydroxybutyrate-co-3-hydroxyalkanoa te) biosynthesis genes in Pseudomonas sp. strain 61-3" JOURNAL OF BACTERIOLOGY, WASHINGTON, DC, US, vol. 180, no. 24, December 1998 (1998-12), pages 6459-6467, XP002167124 ISSN: 0021-9193
- GARCIA BELEN ET AL: "Novel biodegradable aromatic plastics from a bacterial source: Genetic and biochemical studies on a route of the phenylacetyl-CoA catabolon" JOURNAL OF BIOLOGICAL CHEMISTRY, AMERICAN SOCIETY OF BIOLOGICAL CHEMISTS, BALTIMORE, MD, US, vol. 274, no. 41, 8 October 1999 (1999-10-08), pages 29228-29241, XP002176383 ISSN: 0021-9258
- HUISMAN G W ET AL: "METABOLISM OF POLY-3-HYDROXYALKANOATES PHAS BY PSEUDOMONAS-OLEOVORANS IDENTIFICATION AND SEQUENCES OF GENES AND FUNCTION OF THE ENCODED PROTEINS IN THE SYNTHESIS AND DEGRADATION OF PHA" JOURNAL OF BIOLOGICAL CHEMISTRY, vol. 266, no. 4, 1991, pages 2191-2198, XP002176382 ISSN: 0021-9258

## Description

### BACKGROUND OF THE INVENTION

### Field of the Invention

This invention relates to a polyhydroxyalkanoate (hereinafter, referred to as a "PHA") synthase, a gene encoding the PHA synthase, a recombinant vector containing the gene, a transformant capable of expressing the PHA synthase which has been transformed by the recombinant vector, a process for producing the PHA synthase utilizing the transformant, and a process for preparing the PHA utilizing the transformant. In particular, this invention relates to a microorganism-derived PHA synthase capable of producing a polyhydroxyalkanoate and a gene encoding the PHA synthase utilized for expressing the PHA synthase by transformation.

### Related Background Art

There have been reported a number of microorganisms producing poly-3-hydroxybutyric acid (PHB) or another PHA and storing it therein ("Biodegradable Plastic Handbook", edited by Biodegradative Plastic Research Society, NTS Co. Ltd., p.178-197). These polymers may be, as conventional plastics, used for producing a variety of products by, for example, melt-processing. Since they are biodegradable, they have an advantage that they can be completely degraded by microorganisms in the natural environment, and they do not cause pollution due to remaining in the natural environment like many conventional polymer compounds. Furthermore, they are excellently biocompatible, and thus are expected to be used in applications such as a medical soft member.

It is known that a composition and a structure of such a PHA produced by a microorganism may considerably vary depending on the type of a microorganism used for the production, a culture-medium composition and culturing conditions. Investigations have been, therefore, mainly focused on controlling such a composition or structure for the purpose of improving physical properties of a PHA.

For example, Japanese Patent Application Laid-Open Nos. 6-15604, 7-14352 and 8-19227 describe that Alcaligenes eutropus H16 (ATCC No. 17699) and its variants may produce 3-hydroxybutyric acid (3HB) and its copolymer with 3-hydroxyvaleric acid (3HV) with various composition ratios by changing a carbon source during culturing.

Japanese Patent Publication No. 2642937 discloses that PHA in which a monomer unit is 3-hydroxyalkanoate with 6 to 12 carbon atoms may be produced by supplying a non-cyclic aliphatic hydrocarbon as a carbon source to Pseudomonas oleovorans (ATCC No. 29347).

Japanese Patent Application Laid-Open No. 5-74492 discloses methods in which Methylobaterium sp., Paracoccus sp., Alcaligenes sp., and Pseudomonas sp. are contacted with a primary alcohol with 3 to 7 carbon atoms to produce a copolymer of 3HB and 3HV.

Japanese Patent Application Laid-Open Nos. 5-93049 and 7-265065 disclose that Aeromonas caviae is cultured using oleic acid or olive oil as a carbon source to produce a two-component copolymer of 3HB and 3-hydroxyhexanoic acid (3HHx).

Japanese Patent Application Laid-Open No. 9-191893 discloses that Comamonas acidovorans IF013852 is cultured using gluconic acid and 1,4-butanediol as carbon sources to produce a polyester having 3HB and 4-hydroxybutyric acid as monomer units.

Furthermore, it is reported that certain microorganisms produce PHAs having a variety of substituents such as unsaturated hydrocarbon, ester, aryl (aromatic), and cyans groups, halogenated hydrocarbon and epoxide. Recently, there have been attempts for improving physical properties of a PHA produced by a microorganism using such a procedure. For example, Makromol. Chem., 191, 1957-1965 (1990); Macromolecules, 24, 5256-5260 (1991); and Chirality, 3, 492-494 (1991) describe production of a PHA comprising 3-hydroxy-5-phenylvaleric acid (3HPV) as a monomer unit by Pseudomonas oleovorans, where variations in polymer physical properties probably due to the presence of 3HPV were observed.

EP 0 897 005 discloses a polypeptide having polyester synthase activity and comprising a specified amino acid sequence or variants thereof obtained by deletion, replacement or addition of one or more amino acids. Further disclosed are a polyester synthase gene comprising DNA coding for the polypeptide, and a recombinant vector comprising the gene. B. Garcia et al. describe synthesis of polymers containing units of 3-hydroxy-n-phenylalkanoic acids by Pseudomonas putida U (in J. Biol. Chem. 274 (1999), pp29228-29241).
And G. Huisman et al. report on metabolism of poly(3-hydroxyalkanoates) (PHAs) by Pseudomonas oleovorans (in J. Biol. Chem. 266 (1991), pp2191-2198).

As described above, microorganism-produced PHAs with various combinations of composition and structure have been obtained by varying factors such as the type of a microorganism used, a culture medium composition and culturing conditions. Each microorganism has an intrinsic PHA synthase with a substrate specificity which is significantly different from others. Thus, it has been difficult to produce PHAs comprising different monomer units suitable to a variety of applications using known microorganisms or PHA synthases in such known microorganisms.

Meanwhile, as described above, a PHA having a variety of substituents in its side chains may be expected to be a "functional polymer" having significantly useful functions and properties owing to the properties of the introduced substituents. It is, therefore, extremely useful and important to search and develop a microorganism which can produce and store a very useful polymer having both such functionality and biodegradability. Furthermore, identification of a PHA synthase involved in production of the highly useful PHA and obtaining a gene encoding the PHA synthase may allow us to produce a novel transformed microorganism capable of producing a desired PHA. That is, constructing a recombinant vector comprising a gene encoding a PHA synthase and providing a microorganism transformed by the recombinant vector may allow us to prepare a PHA using the transformed microorganism or to express a recombinant type of PHA synthase. As described above, it may be important that a transformed microorganism is used to prepare a desired PHA for providing a highly useful tool for improving a productivity for the PHA and for promoting utilization of the PHA.

### SUMMARY OF THE INVENTION

Objects of this invention which can solve the above problems are to search a novel microorganism capable of producing and storing in microorganisms a PHA having a novel side-chain structure, to identify an enzyme protein related to the ability of producing the novel PHA, i.e., a novel PHA synthase, and to determine a gene encoding its amino acid sequence. More specifically, an object of the present invention is to provide a novel PHA synthase derived from a microorganism producing a PHA having a novel side chain structure and a DNA encoding its amino acid sequence. Another object of this invention is to provide a recombinant vector to which a DNA encoding an available PHA synthase is introduced and which is used for transformation of a microorganism and a transformed microorganism produced using the recombinant vector. A further object of this invention is to provide a process for expressing and producing a recombinant PHA synthase in the transformed microorganism and a process for preparing a desired PHA using the transformed microorganism.

Still another object of this invention is to provide a modified PHA synthase in which its amino acid sequence is modified as long as an enzyme activity is not affected in expression of the recombinant PHA synthase in the transformed microorganism as described above and a DNA encoding the modified amino acid sequence.

The above objects are achieved by the polyhydroxyalkanoate synthase according to claims 1, 2, 5, and 6, the polyhydroxyalkanoate synthase gene according to claims 3, 4, 7, and 8, the recombinant vector according to claim 9, the transformed microorganism according to claim 10, the method for preparing polyhydroxyalkanoate according to claim 11, and the method for producing a polyhydroxyalkanoate synthase according to claim 12.

For developing a PHA having a novel side-chain structure useful as, for example, a device material or a medical material aiming at solving the above problems, the inventors have searched a novel microorganism capable of producing and storing the desired PHA therein. Additionally, the inventors have intensely investigated selected novel microorganisms producing a novel PHA for identifying a PHA synthase involved in production of the novel PHA and for obtaining a gene encoding the PHA synthase. Furthermore, the inventors have conducted investigation for constructing a recombinant vector with a gene for the obtained PHA synthase, transforming a host microorganism using the recombinant vector, expressing a recombinant PHA synthase in the transformed microorganism obtained and determining production of the desired PHA.

In the course of the above investigation, the inventors synthesized 5-(4-fluorophenyl) valeric acid (FPVA) represented by formula (II): 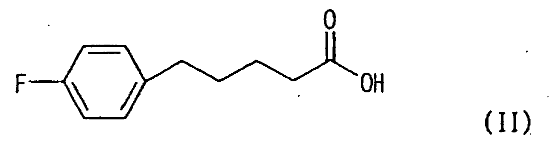 and separated from a soil a novel microorganism capable of converting the above compound (II) as a starting material (substrate) into corresponding 3-hydroxy-5-(4-fluorophenyl)valeric acid (3HFPV) represented by formula (III): 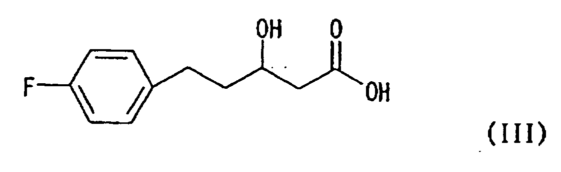 and producing and storing a novel PHA with a monomer unit represented by formula (I): 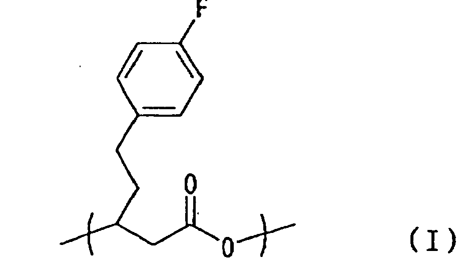 derived from 3HFPV. The novel microorganism separated is designated as H45 strain. The inventors have also found that in addition to the above enzymatic activity for converting FPVA into 3HFPV, the H45 strain may also use 4-cyclohexylbutyric acid (CHxBA) represented by formula (IV): 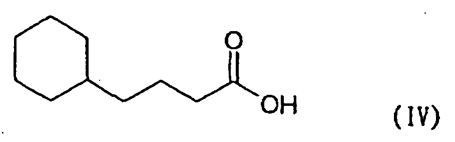 as a starting material (substrate) to convert it into 3-hydroxy-4-cyclohexylbutyric acid (3HCHxB) represented by formula (V): 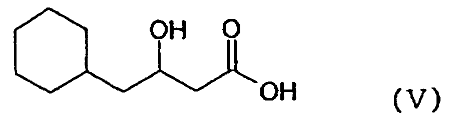 and to produce and store a PHA with a monomer unit represented by formula (VI): 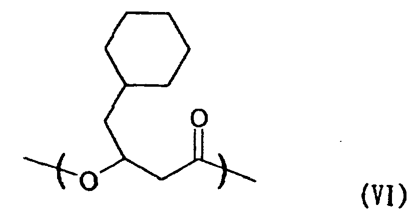 derived from 3HCHxB.

Microbiological properties of H45 strain are as follows.

### <Microbiological properties of H45 strain>

### Morphologic properties

Cell shape and size:
Bacilliform, 0.8 µm × 1.0 to 1.2 µm
Cell polymorphism: No
Motility: Yes
Sporulation: No
Gram stainability: Negative
Colonization: Circular, smooth in the overall periphery, low convex, smooth surface, gloss, yellowish white

### Physiological properties

Catalase: Positive
Oxidase: Positive
O/F test: oxidized form
Reduction of a nitrate: Negative
Indole formation: Negative
Acidification of dextrose: Negative
Arginine dihydrolase: Negative
Urease: Negative
Esculin hydrolysis: Negative
Gelatin hydrolysis: Negative
β-Galactosidase: Negative
Fluorochrome production on King's B agar: Positive
Formation with 4% Nacl: Negative
Accumulation of poly-β-hydroxybutyric acid: Negative

### Substrate assimilation ability

Dextrose: Positive
L-Arabinose: Negative
D-Mannose: Positive
D-Mannitol: Positive
N-Acetyl-D-glucosamine: Positive
Maltose: Negative
Potassium gluconate: Positive
n-Capric acid: Positive
Adipic acid: Negative
dl-Malic acid: Positive
Sodium citrate: Positive
Phenyl acetate: Positive

From these microbiological properties, the inventors have attempted to categorize H45 strain according to Bergey's Manual of Systematic Bacteriology, Volume 1 (1984) and Bergey's Manual of Determinative Bacteriology 9th ed. (1994) to determine that the strain belongs to Pseudomonas cichorii. Thus, it was designated as Pseudomonas cichorii H45. There have been no reports on a strain in Pseudomonas cichorii capable of producing a PHA as exhibited by H45 strain. The inventors have, therefore, determined that P161 strain is a novel microorganism. The applicant deposited Pseudomonas cichorii H45 to Patent Microorganism Depository Center in the National Institute of Bioscience and Human Technology, Agency of Industrial Science and Technology, Ministry of International Trade and Industry, under the deposition number of FERM P-17410. H45 strain has been internationally deposited on the basis of the Budapest Treaty, and its international accession number is "FERM BP-7374".

The inventors achieved cloning a gene for a PHA synthase from the novel microorganism H45 strain and sequenced the gene. The inventors also determined an amino acid sequence for the PHA synthase encoded by the gene. Based on the above observation, the present invention was achieved.

Specifically, a PHA synthase of the present invention is a polyhydroxyalkanoate synthase having an amino acid sequence of SEQ ID NO. 1 or 3. Furthermore, the PHA synthase of the present invention may be a PHA synthase substantially retaining the amino acid sequence of SEQ ID NO. 1 and having a modified amino acid sequence where amino acids are deleted, substituted or added as long as it does not deteriorate an activity as the polyhydroxyalkanoate synthase, or a PHA synthase substantially retaining the amino acid sequence of SEQ ID NO. 3 and having a modified amino acid sequence where amino acids are deleted, substituted or added as long as it does not deteriorate activity as the polyhydroxyalkanoate synthase.

A PHA synthase gene of the present invention is a gene for a polyhydroxyalkanoate synthase comprising a DNA encoding the amino acid sequence of SEQ ID NO. 1 or the sequence of its modified amino acid, or a gene for a polyhydroxyalkanoate synthase comprising a DNA encoding the amino acid sequence of SEQ ID NO. 3 or the sequence of its modified amino acid. Embodiments of a PHA synthase gene of the present invention derived from a genome gene in H45 strain include a PHA synthase gene comprising a DNA sequence of SEQ ID NO. 2 as a DNA encoding the amino acid sequence of SEQ ID NO. 1 and a PHA synthase gene comprising a DNA sequence of SEQ ID NO. 4 as a DNA encoding the amino acid sequence of SEQ ID NO. 3.

This invention also provides a recombinant vector comprising a gene DNA encoding the above amino acid sequence as a polyhydroxyalkanoate synthase gene. This invention also provides a transformed microorganism transformed by introducing a recombinant vector adapted to a host.

The present invention also provides a process for preparing a polyhydroxyalkanoate comprising the steps of culturing the transformed microorganism to which a recombinant vector has been introduced in a culture medium containing a substrate for a polyhydroxyalkanoate synthase and collecting the polyhydroxyalkanoate from the culture preparation. The present invention also provides a process for producing a polyhydroxyalkanoate comprising the steps of culturing the transformed microorganism to which a recombinant vector has been introduced and making the transformed microorganism produce the polyhydroxyalkanoate.

A preferable process for producing a polyhydroxyalkanoate may utilize substrate specificity characteristic of a polyhydroxyalkanoate synthase derived from H45 strain: for example, preparation of a polyhydroxyalkanoate comprising a monomer unit represented by formula (I) derived from 3HFPV utilizing the above transformed microorganism or preparation of a polyhydroxyalkanoate comprising a monomer unit represented by formula (VI) derived from 3HCHxB.

A PHA synthase and a gene encoding the PHA synthase of the present invention are derived from a novel microorganism, Pseudomonas cichorii H45 strain and exhibits such substrate specificity that it selectively produces a PHA comprising a monomer unit having a novel side chain structure. A recombinant vector comprising the PHA synthase gene and a microorganism transformed by the recombinant vector are capable of producing a PHA exhibiting substrate specificity similar to Pseudomonas cichorii H45. Thus, a PHA synthase gene of this invention encodes an enzyme which permits preparation of a PHA selectively comprising a monomer unit having a novel side-chain structure and allows us to create a transformed microorganism useful for preparing a PHA having various useful physical properties which may be expected to be applied to a functional polymer.

### BRIEF DESCRIPTION OF THE DRAWINGS

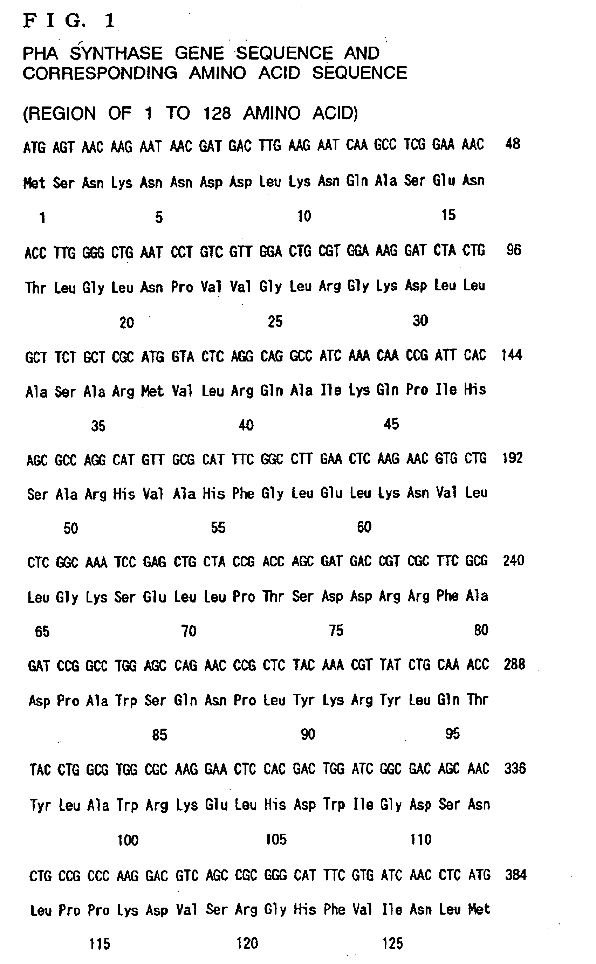

FIG. 1 illustrates a base sequence encoding the first PHA synthase derived from Pseudomonas cichorii H45 (FERM BP-7374) and corresponding amino acid sequence;

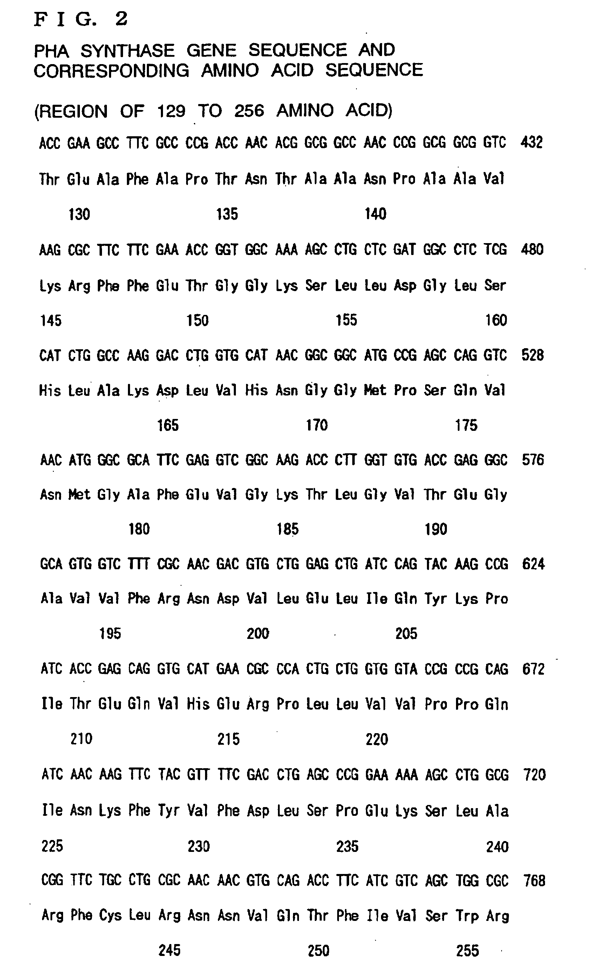

FIG. 2 illustrates a base sequence encoding the first PHA synthase derived from Pseudomonas cichorii H45 (FERM BP-7374) and corresponding amino acid sequence;

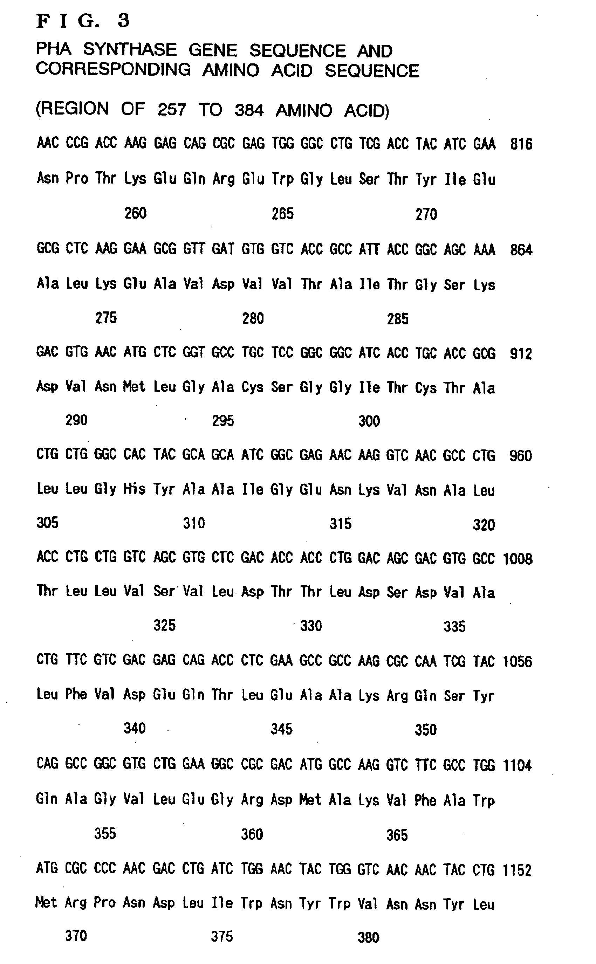

FIG. 3 illustrates a base sequence encoding the first PHA synthase derived from Pseudomonas cichorii H45 (FERM BP-7374) and corresponding amino acid sequence;

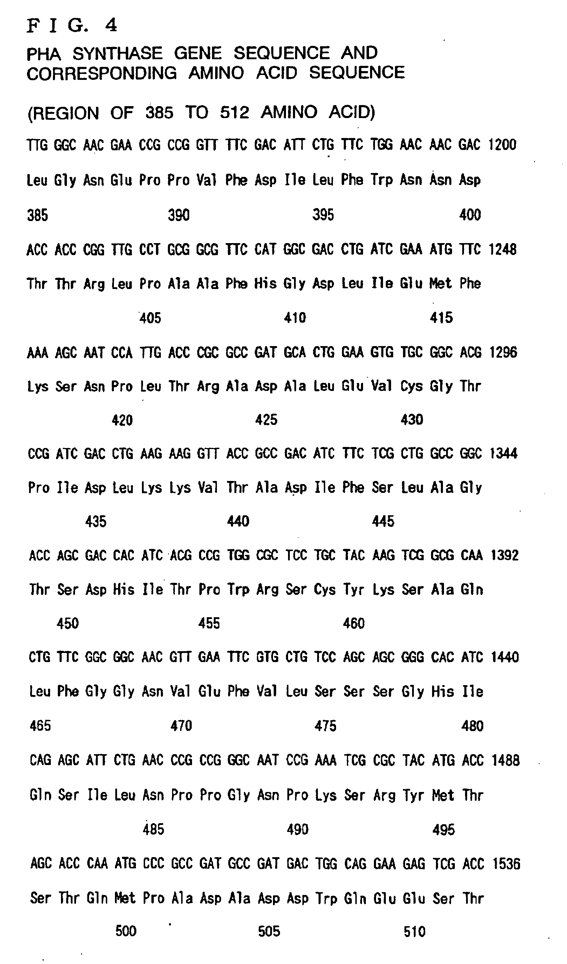

FIG. 4 illustrates a base sequence encoding the first PHA synthase derived from Pseudomonas cichorii H45 (FERM BP-7374) and corresponding amino acid sequence;

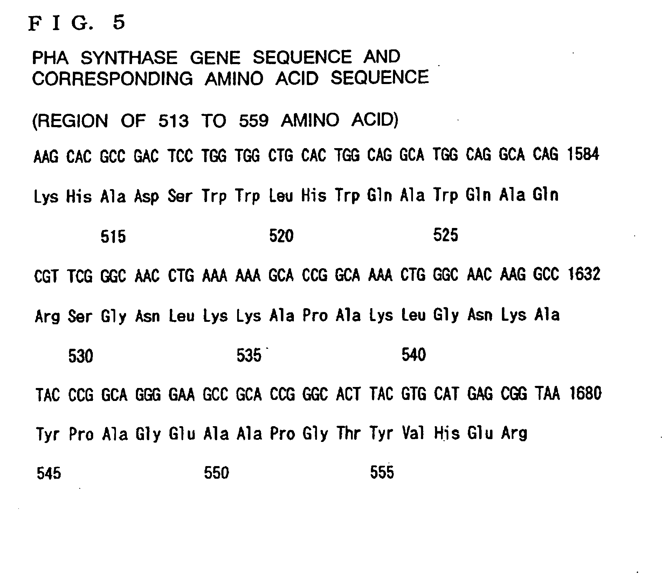

FIG. 5 illustrates a base sequence encoding the first PHA synthase derived from Pseudomonas cichorii H45 (FERM BP-7374) and corresponding amino acid sequence;

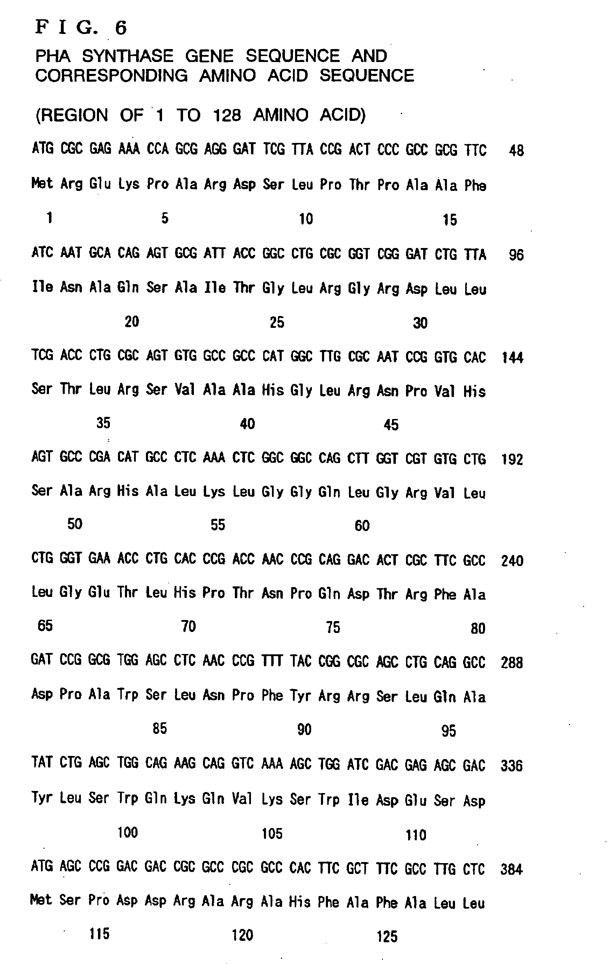

FIG. 6 illustrates a base sequence encoding the second PHA synthase derived from Pseudomonas cichorii H45 (FERM BP-7374) and corresponding amino acid sequence;

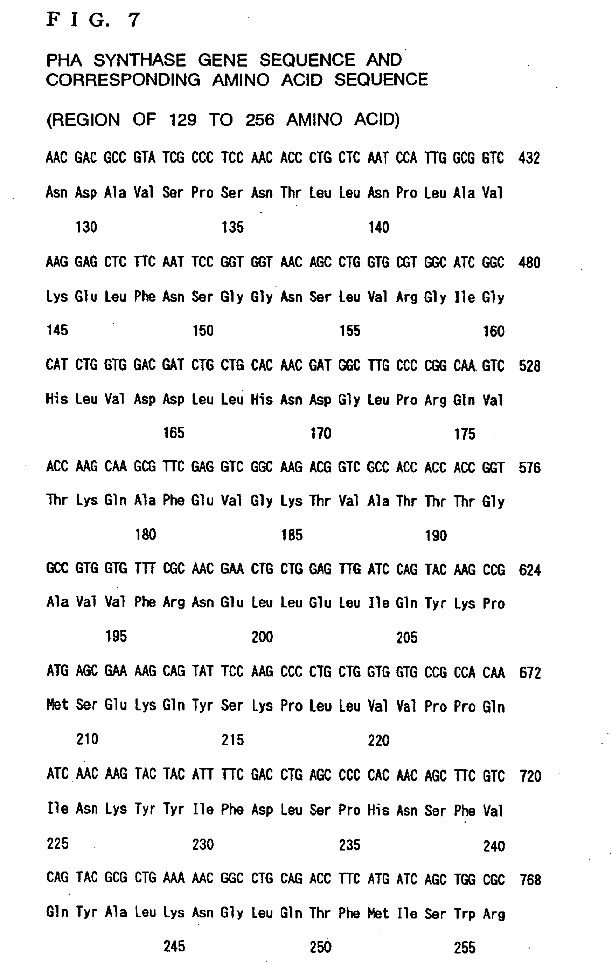

FIG. 7 illustrates a base sequence encoding the second PHA synthase derived from Pseudomonas cichorii H45 (FERM BP-7374) and corresponding amino acid sequence;

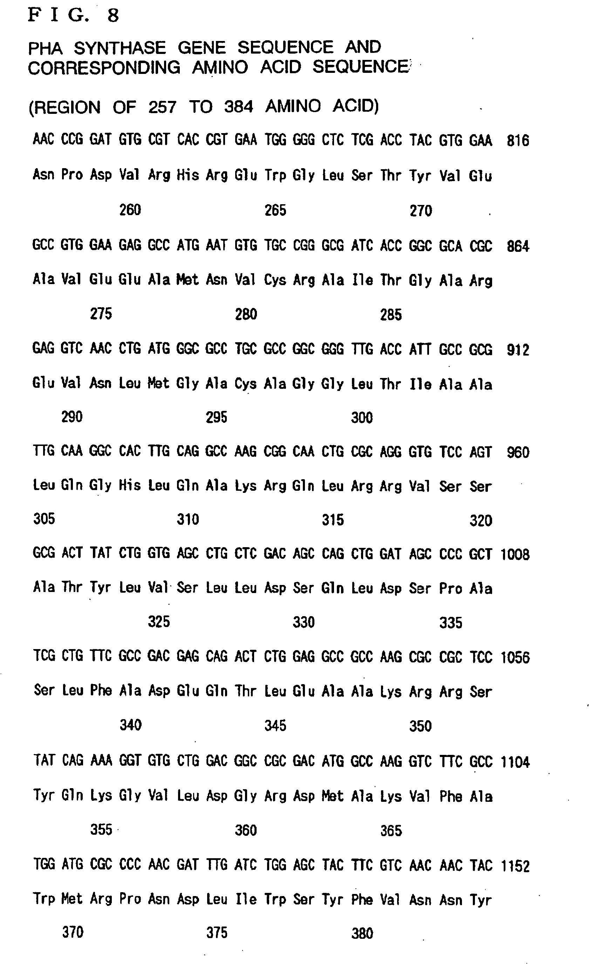

FIG. 8 illustrates a base sequence encoding the second PHA synthase derived from Pseudomonas cichorii H45 (FERM BP-7374) and corresponding amino acid sequence;

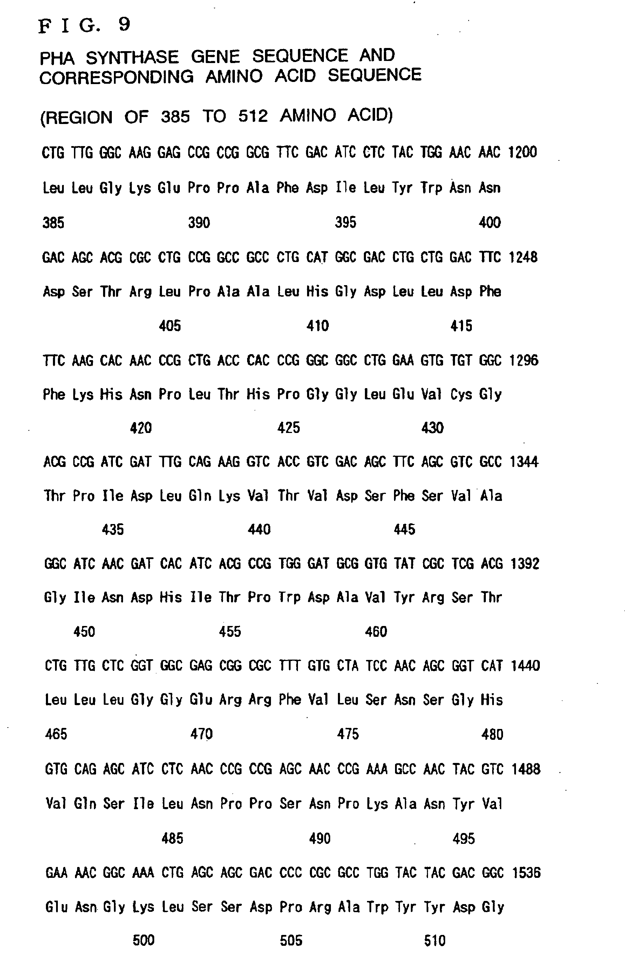

FIG. 9 illustrates a base sequence encoding the second PHA synthase derived from Pseudomonas cichorii H45 (FERM BP-7374) and corresponding amino acid sequence; and

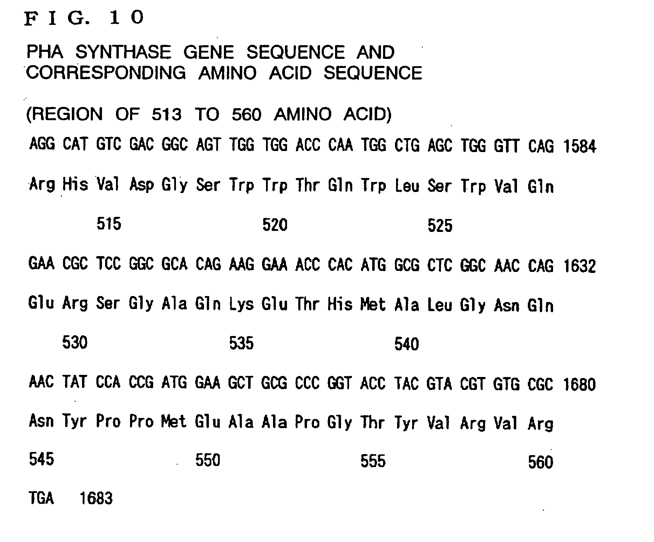

FIG. 10 illustrates a base sequence encoding the second PHA synthase derived from Pseudomonas cichorii H45 (FERM BP-7374) and corresponding amino acid sequence.

### DETAILED DESCRIPTION OF THE PREFERRED EMBODIMENTS

A PHA synthase of this invention is an enzyme protein derived from a novel microorganism isolated by the present inventors, Pseudomonas cichorii H45 (FERM P-17410 FERM BP-7374). Specifically, it can covert 5-(4-fluorophenyl)valeric acid (FPVA) into corresponding 3-hydroxy-5-(4-fluorophenyl)valeric acid (3HFPV) and thus has enzymatic activity involved in production of a PHA comprising a corresponding monomer unit.

A PHA synthase and a gene encoding the enzyme of this invention will be more specifically described.

From H45 strain, the inventors have cloned a gene translated into a PHA synthase which exhibits the above substrate specificity, to determine the presence of a PHA synthase comprising at least two amino acid sequences. Specifically, a PHA synthase of this invention in a chromogene in H45 strain comprises two enzymes, i.e., a PHA synthase comprising the amino acid sequence of SEQ ID NO. 1 encoded by a DNA having the sequence of SEQ ID NO. 2 and a PHA synthase comprising the amino acid sequence of SEQ ID NO. 3 encoded by a DNA having the sequence of SEQ ID NO. 4. Gene DNAs of the sequences of SEQ ID NOs. 2 and 4 may be cloned by the following procedure.

Since a PHA synthase is an enzyme protein translated from a chromogene in Pseudomonas cichorii H45 strain, a chromosome DNA containing a desired PHA synthase is first obtained. A chromosome DNA may be separated from H45 strain cells by a known separation method. For example, H45 strain is cultured in a LB medium or an M9 medium supplemented with an appropriate carbon source, disrupt and treated as described by, for example, Marmer et al. in Journal of Molecular Biology, Vol. 3, p. 208 (1961) to prepare a chromosome DNA.

Then, a gene library is prepared from the chromosome DNA thus obtained. The chromosome DNA is degraded using an appropriate restriction enzyme (e.g., Sau3AI) and a fragment with a proper length is ligated with a ligatable vector truncated with a restriction enzyme (e.g., BamHI) to prepare a gene library.

Depending on a vector used in preparing a library, a proper fragment length varies, e.g., about 4000 to 25000 bps for a usual plasmid vector and about 15000 to 30000 bps for a cosmid or phage vector. A proper length of DNA fragment may be collected by a known method such as a method using a sucrose density gradient or using an agarose gel described in Molecular Cloning, Cold Spring Harbor Laboratory (1982).

Since E. coli is used as a host microorganism in a gene library, a vector is a phage vector or plasmid vector which can autonomously grow in the host microorganism (E. coli). Examples of phage or cosmic vectors generally used include pWE15, M13, λEMBL3, λEMBL4, λFIXII, λDASHII, λZAPII, λgt10, λgt11, Charon4A and Charon21A. Examples of frequently used plasmid vectors include pBR, pUC, pBluescriptII, pGEM, pTZ and pET groups. In addition to E. coli, various shuttle vectors may be used, e.g., vectors which may autonomously replicate in a plurality of host microorganisms such as Pseudomonas sp. Again, these vectors may be, depending on a chromosome DNA to be ligated to them, truncated with a proper restriction enzyme to provide a desired fragment.

A chromosome DNA fragment may be ligated with a vector fragment using a DNA ligase. For example, a commercially available ligation kit (Takara Shuzo Co., Ltd., etc.) may be used. Thus, for example, various chromosome DNA fragments may be ligated with a plasmid vector fragment to prepare a mixture of recombinant plasmids comprising various DNA fragments (hereinafter, referred to as a "gene library").

In addition to a method using a proper length of chromosome DNA fragment, a gene library may be prepared by a method that all mRNAs are extracted from H45 strain, purified and used for preparation of a cDNA fragment using a reverse transcriptase as described in Molecular Cloning, Cold Spring Harbor Laboratory, 1982. Alternatively, a prepared vector is used in a gene library to transform or transduce to E. coli, and then the host E. coli is cultured to amplify the gene library to a large amount as described in Molecular Cloning, Cold Spring Harbor Laboratory, 1982.

A recombinant vector comprising a gene DNA fragment may be introduced into a host microorganism by a known method. For example, when using E. coli as a host microorganism, a recombinant plasmid vector may be introduced using a calcium chloride method (Journal of Molecular Biology, Vol. 53, p. 159 (1970)), a rubidium chloride method (Methods in Enzymology, Vol. 68, p. 253 (1979)), electroporation (Current Protocols in Molecular Biology, Vol. 1, p. 1.8.4 (1994)). When using a cosmid vector or phage vector, transduction in a host E. coli may be conducted using in vitro packaging (Current Protocols in Molecular Biology, Vol. 1, p. 5.7.1 (1994)). Alternatively, conjugational transfer with a strain retaining a recombinant vector may be utilized to prepare a strain retaining a vector.

Then, from the gene library, a probe is prepared for obtaining a DNA fragment comprising a PHA synthase gene of H45 strain.

Some base sequences have been reported for PHA synthase genes in known microorganisms; for example, Peoples, O. P. and Sinskey, A. J., J. Biol. Chem., 264, 15293 (1989); Huisman, G. W. et al., J. Biol. Chem., 266, 2191 (1991); Pieper, U. et al., FEMS Microbiol. Lett., 96, 73 (1992); Timm, A. and Steinbuchel, A., Eur. J. Biochem., 209, 15(1992); Matsusaki, H. et al., J. Bacteriol., 180, 6459 (1998). These reported sequences are compared to select a region where a sequence is preserved to a higher degree and thus to design an oligonucleotide for a primer used in polymerase chain reaction (hereinafter, referred to as "PCR"). Such oligonucleotides for a primer utilizing a common feature of PHA synthase genes include, but not limited to, a sequence described in Timm, A. and Steinbuchel, A., Eur. J. Biochem., 209, 15 (1992). An oligonucleotide may be synthesized using, for example, a commercially available DNA synthesizer such as Custom Synthesis Service, Amersham-Pharmacia Biotech, depending on a designed sequence.

For a PHA synthase gene derived from H45 of this invention, synthetic DNAs having the sequences of SEQ ID NOs. 5 and 6 were designed.

Then, the designed oligonucleotide as a primer is subject to polymerase chain reaction (PCR) using a chromosome DNA in H45 strain as a template to obtain a PCR amplified fragment. The PCR amplified fragment, which is derived from the primer, comprises a sequence common in PHA synthase genes at both ends. A partial sequence derived from the PHA synthase gene itself in H45 strain as a template is contained between sequences complementary to the primer at both ends.

The PCR amplified fragment obtained is, therefore, almost 100 % homologous to the PHA synthase gene in H45 strain and is expected to exhibit a higher S/N ratio as a probe in colony hybridization. In addition, it may facilitate stringency control of hybridization.

The above PCR amplified fragment is labeled with an appropriate reagent and used as a probe to colony-hybridize the above chromosome DNA library for selecting a recombinant E. coli strain retaining the PHA synthase gene (Current Protocols in Molecular Biology, Vol. 1, p. 6.0.3 (1994)). For example, the PCR amplified fragment may be labeled using a common detection system using a labeled enzyme or a commercially available kit such as AlkPhosDirect (Amersham-Pharmacia Biotech).

A recombinant E. coli strain retaining a gene fragment comprising a PHA synthase gene may be selected by, in addition to the above method using a gene type, a method using a phenotype where PHA synthesis is directly evaluated. Specifically, in expression of a PHA synthase from a retained PHA synthase gene in a recombinant E. coli strain, PHA is produced by the PHA synthase. PHA synthesis may be detected to select a recombinant E. coli strain in which the PHA synthase is expressed. PHA synthesis may be detected by, for example, staining with Sudan Black B (Archives of Biotechnology, Vol. 71, p. 283 (1970)) or determination of PHA accumulation by phase contrast microscopy.

A plasmid is collected from a recombinant E. coli selected by any of the above methods using an alkali method (Current Protocols in Molecular Biology, Vol. 1, p. 1.6.1 (1994)). The collected plasmid may be used to provide a DNA fragment comprising a PHA synthase gene or multiple DNA fragments partially containing a PHA synthase gene. The DNA fragment obtained may be sequenced by, for example, the Sanger's sequencing method (Molecular Cloning, Vol. 2, p. 13.3 (1989). Specifically, it may be conducted by a dye-primer method or a dye-terminator method using an automatic sequencer such as DNA Sequencer 377A (Parkin Elmer). Since the sequence of the vector itself in which the DNA fragment has been incorporated is known, the sequence of the DNA fragment cloned therein may be unequivocally analyzed.

After sequencing all the obtained DNA fragments comprising a PHA synthase gene, hybridization may be conducted using a DNA fragment prepared by an appropriate method such as chemical synthesis, PCR using a chromosome DNA as a template or degradation of a DNA fragment comprising the sequence with a restriction enzyme as a probe to provide a PHA synthase gene DNA of this invention.

The inventors have selected a gene translated into a PHA synthase exhibiting the above substrate specificity from H45 strain according to the above procedure to find a PHA synthase comprising at least two amino acid sequences. Specifically, the inventors have found a PHA synthase gene collected from the chromosome DNA of H45 strain and comprising the sequence of SEQ ID NO. 2 and a PHA synthase encoded by the gene and comprising the amino acid of SEQ ID NO. 1 as well as a PHA synthase gene comprising the sequence of SEQ ID NO. 4 and a PHA synthase encoded by the gene and comprising the amino acid of SEQ ID NO. 3.

A PHA synthase gene of the present invention may include a degenerated isomer encoding the same polypeptide which has the same amino acid sequence and is different in a degeneration codon. More specifically, it also includes a degenerated isomer by selection and conversion of a more frequently used degenerated codon encoding the same amino acid depending on a host. Besides the PHA synthase comprising the amino acid sequence of SEQ ID NO. 1 inherent in H45 strain and the PHA synthase comprising the amino acid sequence of SEQ ID NO. 3, a PHA synthase of this invention may have mutation such as deletion, substitution and addition for several amino acids as long as its PHA producing activity and substrate specificity may not be deteriorated or the amino acid sequence may be maintained. Mutation such as deletion, substitution and addition may be introduced by a site mutation introduction technique based on a PHA synthase gene inherent in H45 strain having the sequence of SEQ ID NO. 2 or 4 (Current Protocols in Molecular Biology Vol. 1, p. 8.1.1 (1994)).

A recombinant vector of the present invention is used in an application where a recombinant PHA synthase of this invention is expressed using Pseudomonas sp. or a microorganism such as E. coli as a host. It is, therefore, preferable that the recombinant vector of this invention itself can autonomously replicate in a host used while comprising a promoter for expression, a PHA synthase gene DNA of this invention and a transcription termination sequence suitable to the host. In addition, it is preferable that after introducing the recombinant vector, a vector comprising various marker genes used for its selection is used.

Expression vectors suitable to various types of bacterial hosts such as Pseudomonas sp. and E. coli include pLA2917 (ATCC 37355) having a RK2 replication origin which may be replicated and retained by a range of hosts or pJRD215 (ATCC 37533) having a RSF1010 replication origin. Without being limited to these, any vector having a replication origin which may be replicated and retained by a range of hosts may be used. Any promoter which may be expressed in a bacterium as a host may be used; for example, promoters derived from E. coli, a phage, etc. such as trp, trc, tac, lac, PL, PR, T7 and T3 promoters.

When using a yeast as a host, an expression vector may be YEp13, YCp50, pRS or pYEX vector. A promoter may be, for example, GAL or AOD promoter.

A transformed microorganism of this invention may be produced by introducing a recombinant vector of this invention into a host suitable to an expression vector used during preparing the recombinant vector. Examples of bacteria which may be used as a host include Esherichia sp., Pseudomonas sp., Ralstonia sp., Alcaligenes sp., Comamonas sp., Burkholderia sp., Agrobacterium sp., Flabobacterium sp., Vibrio sp., Enterobacter sp., Rhizobium sp., Gluconobacter sp., Acinetobacter sp., Moraxella sp., Nitrosomonas sp., Aeromonas sp., Paracoccus sp., Bacillus sp., Clostridium sp., Lactobacillus sp., Corynebacterium sp., Arthrobacter sp., Achromobacter sp., Micrococcus sp., Mycobacterium sp., Streptococcus sp., Streptomyces sp., Actinomyces sp., Norcadia sp. and Methylobacterium sp. A recombinant DNA may be introduced into a bacterium by an appropriate technique such as the above calcium chloride method and electroporation.

Besides the above bacteria, yeasts and molds such as Saccharomyces sp. and Candida sp. may be used as a host. A recombinant DNA may be introduced into an yeast by, for example, electroporation (Methods Enzymol., 194, 182-187 (1990)), a spheroplast method (Proc. Natl. Acad. Sci. USA, 84, 1929-1933 (1978)) and a lithium acetate method (J. Bacteriol., 153, 163-168 (1983)).

A PHA synthase of this invention may be prepared by culturing a transformant of this invention prepared by the above procedure and making a corresponding PHA synthase gene in an introduced expression vector producing the synthase as a recombinant protein. The PHA synthase of this invention is produced and accumulated in the culture (cultured bacterium or culture supernatant) and separated from the culture to be used for production of a recombinant enzyme protein. For this purpose, a transformant of this invention may be cultured by a usual procedure used for culturing a host. Culturing may be conducted by any of common methods used for culturing a microorganism such as batch, flow batch, continuous culturing and reactor styles. This culturing may be conducted by using, for example, a medium containing an inducer for expressing the above polyhydroxyalkanoate synthase gene.

For a transformant obtained using a bacterium such as E. coli as a host, a medium used for culturing may be a complete medium or synthetic medium such as LB medium and M9 medium. A microorganism may be grown by aerobically culturing at a culturing temperature of 25 to 37°C for 8 to 72 hours. Then, the bacteria are collected for obtaining a PHA synthase accumulated in them. Examples of a carbon source for the microorganism include sugars such as glucose, fructose, sucrose, maltose, galactose and starches; lower alcohols such as ethanol, propanol and butanol; polyalcohols such as glycerol; organic acids such as acetic acid, citric acid, succinic acid, tartaric acid, lactic acid and gluconic acid; and aliphatic acids such as propionic acid, butanoic acid, pentanoic acid, hexanoic acid, heptanoic acid, octanoic acid, nonanoic acid, decanoic acid, undecanoic acid and dodecanoic acid.

Examples of a nitrogen source include ammonia; ammonium salts such as ammonium chloride, ammonium sulfate and ammonium phosphate; and natural product derivatives such as peptone, meat extract, yeast extract, malt extract, casein decomposition products and corn steep liquor. Examples of an inorganic material include potassium dihydrogen phosphate, potassium monohydrogen phosphate, magnesium phosphate, magnesium sulfate and sodium chloride. The culture medium may contain an antibiotic such as kanamycin, ampicillin, tetracyclin, chloramphenicol and streptomycin, depending on, for example, the type of a drug resistance gene used as a marker gene.

When using an inducible promoter in an expression vector, expression may be enhanced by adding a proper inducer depending on the type of the promoter during culturing a transformed microorganism. For example, the inducer may be isopropyl-β-D-thiogalactopyranoside (IPTG), tetracyclin or indoleacrylic acid (IAA).

A PHA synthase may be separated and purified by centrifuging and collecting a culture obtained and processing it by a technique such as affinity chromatography, cation or anion exchange chromatography and gel filtration alone or in combination as appropriate. Whether a purified material is a desired enzyme is determined by a usual method such as SDS polyacrylamide gel electrophoresis and Western blotting.

This invention is not limited to the procedures as described above for culturing of a transformed microorganism of the present invention, production of a PHA synthase by the transformed microorganism of this invention and accumulating it in bacterial cells, and collection and purification of the PHA synthase from the cells.

A transformed microorganism of the present invention may be used for expressing a recombinant PHA synthase to produce a desired PHA by culturing thereof. For example, the microorganism may be cultured under the above culturing conditions to produce a recombinant PHA synthase while a substrate corresponding to the desired PHA on which the PHA synthase acts is added to a medium. Most conveniently, the PHA may be collected from the culture and the producing bacteria by extraction with an organic solvent commonly used such as chloroform. In an environment where using an organic solvent such as chloroform is undesirable, the culture may be treated a surfactant such as SDS, an enzyme such as lysozyme, or an agent such as EDTA, sodium hypochlorite and ammonia to remove bacterium components other than the PHA for collecting the PHA. This invention is not limited to the above procedures for culturing of a transformed microorganism of this invention for production of a PHA, production of a PHA by and accumulation thereof in a cultured microorganism, and collection of the PHA from a recombinant microorganism.

### Examples

This invention will be more specifically described with reference to Examples, although these Examples are illustrated as the best embodiments of this invention and do not limit the technical range of this invention.

### (Example 1) Cloning of a PHA synthase gene of H45 strain.

H45 strain was cultured in 100 mL of LB medium (1 % polypeptone, 0.5 % yeast extract, 0.5 % sodium chloride, pH 7.4) at 30°C overnight and then a chromosome DNA was separated and collected as described by Marmer. The obtained chromosome DNA was completely digested using a restriction enzyme BglII. A vector pUC18 was cleaved with a restriction enzyme BamHI. After dephosphorylation of the terminals (Molecular Cloning, Vol. 1, p. 5.7.2 (1989), Cold Spring Harbor Laboratory), the cleaved site of the vector (cloning site) and the chromosome DNA fragment after BglII complete digestion were ligated using a DNA ligation kit Ver. II (Takara Shuzo Co., Ltd.). The plasmid vector in which the chromosome DNA fragment was integrated was used to transform Escheichia coli HB101 for preparing a chromosome DNA library for H45 strain.

Then, in order to select a DNA fragment comprising a PHA synthase gene of H45 strain, a probe for colony hybridization was prepared. An oligonucleotide consisting of the sequences of SEQ ID NOs. 5 and 6 (Amersham-Pharmacia Biotech) was prepared and used as a primer for PCR using the chromosome DNA as a template. A PCR-amplified DNA fragment was used as a probe. Labeling of the probe was conducted using a commercially available labeling enzyme system AlkPhosDirect (Amersham-Pharmacia Biotech). The labeled probe thus obtained was used to select an E. coli strain containing a recombinant plasmid comprising the PHA synthase gene from the chromosome DNA library of H45 strain by colony hybridization. From the selected strain, the plasmid was collected by an alkali method to prepare a DNA fragment comprising a PHA synthase gene.

The gene DNA fragment thus obtained was recombined in a vector pBBR122 (Mo BiTec) comprising a wide host range of replication region which did not belong to IncP, IncQ or IncW in an incompatible group. The recombinant plasmid was transformed in Pseudomonas cichorii H45ml strain (a strain depleted of PHA synthesizing ability) by electroporation, and then the H45m1 strain regained PHA synthesizing ability and exhibited complementarity. It demonstrates that the selected gene DNA fragment comprises a region of a PHA synthase gene translatable into a PHA synthase in Pseudomonas cichorii H45ml.

The DNA fragment comprising a PHA synthase gene was sequenced by the Sanger's sequencing method. It was thus found that the determined sequence comprised the sequences of SEQ ID NOs. 2 and 4 each of which encoded a peptide chain. As described below, it was determined that both proteins consisting of a peptide chain had enzyme activity and that the sequences of SEQ ID NOs. 2 and 4 were therefore PHA synthase genes. Specifically, it was found that the sequences of SEQ ID NOs. 2 and 4 encoded the amino acid sequences of SEQ ID NOs. 1 and 3, respectively, and that a protein comprising one of these amino acid sequences alone could produce a PHA.

### (Example 2) Recombination of a PHA synthase gene of H45 strain to an expression vector.

A PHA synthase gene having the sequence of SEQ ID NO. 2 was PCRed using a chromosome DNA as a template to reproduce the whole length of a PHA synthase gene. An oligonucleotide having a sequence which was an upstream primer to the sequence of SEQ ID NO. 2 and had a sequence upstream of its initiation codon (SEQ ID NO. 7) and an oligonucleotide having a sequence which was a downstream primer to the sequence of SEQ ID NO. 2 and had a sequence downstream of its termination codon (SEQ ID NO. 8) were designed and prepared (Amersham-Pharmacia Biotech). Using these oligonucleotides as a primer, PCR was conducted to amplify the whole length of the PHA synthase gene (LA-PCR kit; Takara Shuzo Co., Ltd.).

Likewise, a PHA synthase gene having the sequence of SEQ ID NO. 4 was PCRed using a chromosome DNA as a template to reproduce the whole length of a PHA synthase gene. An oligonucleotide having a sequence which was an upstream primer to the sequence of SEQ ID NO. 4 and had a sequence upstream of its initiation codon (SEQ ID NO. 9) and an oligonucleotide having a sequence which was a downstream primer to the sequence of SEQ ID NO. 4 and had a sequence downstream of its termination codon (SEQ ID NO. 10) were designed and prepared (Amersham-Pharmacia Biotech). Using these oligonucleotides as a primer, PCR was conducted to amplify the whole length of the PHA synthase gene (LA-PCR kit; Takara Shuzo Co., Ltd.).

Each of the obtained PCR amplified fragment containing the whole length of the PHA synthase gene was completely digested using a restriction enzyme HindIII. Separately, an expression vector pTrc99A was also truncated with a restriction enzyme HindIII and dephosphorylated (Molecular Cloning, Vol. 1, p. 5.7.2 (1989), Cold Spring Harbor Laboratory). To the truncated site of the expression vector pTrc99A was ligated the DNA fragment comprising the whole length of the PHA synthase gene from which unnecessary sequences had been removed at both ends, using a DNA ligation kit Ver. II (Takara Shuzo Co., Ltd.).

Using the recombinant plasmids obtained, Escherichia coli HB101 (Takara Shuzo Co., Ltd.) was transformed by a calcium chloride method. The recombinants obtained were cultured, and the recombinant plasmids were amplified and collected individually. The recombinant plasmid retaining the gene DNA of SEQ ID NO. 2 was designated pH45-C1 (derived from SEQ ID NO. 2) while the recombinant plasmid retaining the gene DNA of SEQ ID NO. 4 was designated pH45-C2 (derived from SEQ ID NO. 4).

### (Example 3) PHA production (1) using a PHA synthase gene recombinant E. coli.

Using the recombinant plasmids obtained in Example 2, pH45-C1 (derived from SEQ ID NO. 2) and pH45-C2 (derived from SEQ ID NO. 4), an Escherichia coli HB101fB (fadB deficient strain) was transformed by a calcium chloride method to prepare recombinant E. coli strains retaining the recombinant plasmid, pH45-C1 and pH45-C2 recombinant strains, respectively.

Each of the pH45-C1 and pH45-C2 recombinant strains was inoculated to 200 mL of M9 medium containing 0.5 % yeast extract and 0.1 % FPVA, and the medium was shaken at 37°C with a rate of 125 strokes/min. After 24 hours, the cells were collected by centrifugation, washed once with cold methanol and lyophilized.

The lyophilized pellet was suspended in 100 mL of chloroform and the suspension was stirred at 60°C for 20 hours to extract a PHA. After filtering the extract through a membrane filter with a pore size of 0.45 µm, the filtrate was concentrated by rotary evaporation. Then, the concentrate was re-suspended in cold methanol and the precipitant was collected and dried in vacuo to provide a PHA. The PHA thus obtained was subject to methanolysis as usual and analyzed using a gas chromatography-mass spectrometry apparatus (GC-MS, Shimadzu QP-5050, EI technique) to identify methyl-esterified PHA monomer units. Table 1 shows together a cell dry weight, a polymer dry weight for a collected PHA, a polymer yield per a cell (polymer dry weight/cell dry weight) and identities of monomer units for each strain.

**Table 1**

| | pH45-C1 recombinant strain | pH45-C2 recombinant strain |
|---|---|---|
| Cell dry weight | 730 mg/L | 770 mg/L |
| Polymer dry weight | 36 mg/L | 39 mg/L |
| Polymer dry weight/Cell dry weight | 5 % | 5 % |
| Monomer unit composition (area ratio) | | |
| 3-Hydroxybutyric acid | 0 % | 0 % |
| 3-Hydroxyvaleric acid | 0 % | 0 % |
| 3-Hydroxyhexanoic acid | 0 % | 0 % |
| 3-Hydroxyheptanoic acid | 1 % | 1 % |
| 3-Hydroxyoctanoic acid | 2 % | 3 % |
| 3-Hydroxynonanoic acid | 0 % | 1 % |
| 3-Hydroxydecanoic acid | 4 % | 5 % |
| 3-Hydroxy-5-(4-fluorophenyl) valeric acid | 93 % | 90 % |

These results show that both pH45-Cl and pH45-C2 recombinant strains produce, from the substrate 5-(4-fluorophenyl) valeric acid, PHAs comprising a monomer unit represented by formula (I) derived from corresponding 3-hydroxy-5-(4-fluorophenyl) valeric acid as a main component. It is, therefore, demonstrated that although the pH45-C1 and pH45-C2 recombinant strains exclusively produce PHA synthases having the amino acid sequences of SEQ ID NOs. 1 and 3 translated from the PHA synthase genes comprising the sequences of SEQ ID NOs. 2 and 4, respectively, both strains similarly convert the substrate 5-(4-fluorophenyl) valeric acid into the monomer unit represented by formula (I) derived from corresponding 3-hydroxy-5-(4-fluorophenyl) valeric acid and produce a PHA containing the monomer unit.

## Claims

1. A polyhydroxyalkanoate synthase having the amino acid sequence of SEQ ID NO. 1.

2. A polyhydroxyalkanoate synthase having an amino acid sequence wherein the amino acid sequence of SEQ ID NO. 1 has been modified by deleting, substituting or adding amino acids to such extent that the resulting polyhydroxyalkanoate synthase still has the activity to convert 5(4-fluorophenyl)valeric acid into a polymer comprising 3-hydroxy-5-(4-fluorophenyl)valeric acid as a monomer unit.

3. A polyhydroxyalkanoate synthase gene comprising a DNA sequence encoding the amino acid sequence of the polyhydroxyalkanoate synthase according to claim 2.

4. The polyhydroxyalkanoate synthase gene comprising a DNA sequence of SEQ ID NO. 2 encoding the amino acid sequence of SEQ ID NO. 1.

5. A polyhydroxyalkanoate synthase having the amino acid sequence of SEQ ID NO. 3.

6. A polyhydroxyalkanoate synthase having an amino acid sequence wherein the amino acid sequence of SEQ ID NO. 3 has been modified by deleting, substituting or adding amino acids to such extent that the resulting polyhydroxyalkanoate synthase still has the activity to convert 5(4-fluorophenyl)valeric acid into a polymer comprising 3-hydroxy-5-(4-fluorophenyl)valeric acid as a monomer unit.

7. A polyhydroxyalkanoate synthase gene comprising a DNA sequence encoding the amino acid sequence of the polyhydroxyalkanoate synthase according to claim 6.

8. The polyhydroxyalkanoate synthase gene comprising a DNA sequence of SEQ ID NO. 4 encoding the amino acid sequence of SEQ ID NO, 3.

9. A recombinant vector comprising the gene according to claim 3, 4, 7 or 8 as a polyhydroxyalkanoate synthase gene.

10. A transformed microorganism transformed by introduction of the recombinant vector according to claim 9.

11. A method for preparing a polyhydroxyalkanoate comprising the steps of culturing the transformed microorganism according to claim 10 in a medium containing a substrate for a polyhydroxyalkanoate synthase and isolating the polyhydroxyalkanoate from the culture obtained.

12. A method for producing a polyhydroxyalkanoate synthase comprising the steps of culturing the transformed microorganism according to claim 10 and making the transformed microorganism produce the polyhydroxyalkanoate synthase.

## Patentansprüche

1. Polyhydroxyalkanoat-Synthase, die die Aminosäuresequenz der Sequenzidentifizierungsnr. 1 aufweist.

2. Polyhydroxyalkanoat-Synthase mit einer Aminosäuresequenz, wobei
die Aminosäuresequenz der Sequenzidentifizierungsnr. 1 modifiziert worden ist, indem Aminosäuren so weit deletiert, substituiert oder hinzugefügt wurden, dass die entstehende Polyhdydroxyalkanoat-Synthase noch immer die Aktivität hat, 5(4-Fluorphenyl)valeriansäure in ein Polymer zu überführen, das 3-Hydroxy-5-(4-fluorphenyl)valeriansäure als eine Monomereinheit aufweist.

3. Polyhydroxyalkanoat-Synthase-Gen, umfassend:
eine DNA-Sequenz, die die Aminosäuresequenz der Polyhydroxyalkanoat-Synthase nach Anspruch 2 codiert.

4. Polyhydroxyalkanoat-Synthase-Gen, umfassend:
eine DNA-Sequenz der Sequenzidentifizierungsnr. 2, die die Aminosäuresequenz der Sequenzidentifizierungsnr. 1 codiert.

5. Polyhydroxyalkanoat-Synthase
mit der Aminosäuresequenz der Sequenzidentifizierungsnr. 3.

6. Polyhydroxyalkanoat-Synthase mit einer Aminosäuresequenz, wobei
die Aminosäuresequenz der Sequenzidentifizierungsnr. 3 modifiziert worden ist, indem Aminosäuren so weit deletiert, substituiert oder hinzugefügt wurden, dass die entstehende Polyhdydroxyalkanoat-Synthase noch immer die Aktivität hat, 5(4-Fluorphenyl)valeriansäure in ein Polymer zu überführen, das 3-Hydroxy-5-(4-fluorphenyl)valeriansäure als eine Monomereinheit aufweist.

7. Polyhydroxyalkanoat-Synthase-Gen, umfassend:
eine DNA-Sequenz, die die Aminosäuresequenz der Polyhydroxyalkanoat-Synthase nach Anspruch 6 codiert.

8. Polyhydroxyalkanoat-Synthase-Gen, umfassend:
eine DNA-Sequenz der Sequenzidentifizierungsnr. 4, die die Aminosäuresequenz der Sequenzidentifizierungsnr. 3 codiert.

9. Rekombinant-Vektor, umfassend:
das Gen nach Anspruch 3, 4, 7, oder 8 als ein Polyhydroxyalkanoat-Synthase-Gen.

10. Transformierter Mikroorganismus,
der durch Einführen des Rekombinant-Vektors nach Anspruch 9 transformiert ist.

11. Verfahren zum Herstellen eines Polyhydroxyalkanoats, umfassend die Schritte:
- Züchten des transformierten Mikroorganismus nach Anspruch 10 in einem Medium, das ein Substrat für eine Polyhydroxyalkanoat-Synthase enthält, und
- Abtrennen des Polyhydroxyalkanoats von der erhaltenen Kultur.

12. Verfahren zum Herstellen einer Polyhydroxyalkanoat-Synthase, umfassend die Schritte:
- Züchten des transformierten Mikroorganismus nach Anspruch 10 und
- Bewirken, dass der transformierte Mikroorganismus die Polyhydroxyalkanoat-Synthase produziert.

## Revendications

1. Polyhydroxyalcanoate-synthase ayant la séquence d'aminoacides de la SEQ ID N° 1.

2. Polyhydroxyalcanoate-synthase ayant une séquence d'aminoacides dans laquelle la séquence d'aminoacides de la SEQ ID N° 1 a été modifiée par délétion, substitution ou addition d'aminoacides de telle sorte que la polyhydroxyalcanoate-synthase résultante ait encore l'activité de conversion de l'acide 5(4-fluorophényl)valérique en un polymère comprenant de l'acide 3-hydroxy-5-(4-fluorophényl)-valérique comme motif monomère.

3. Gène de polyhydroxyalcanoate-synthase comprenant une séquence d'ADN codant pour la séquence d'aminoacides de la polyhydroxyalcanoate-synthase suivant la revendication 2.

4. Gène de polyhydroxyalcanoate-synthase comprenant une séquence d'ADN de la SEQ ID N° 2 codant pour la séquence d'aminoacides de la SEQ ID N° 1.

5. Polyhydroxyalcanoate-synthase ayant la séquence d'aminoacides de la SEQ ID N° 3.

6. Polyhydroxyalcanoate-synthase ayant une séquence d'aminoacides dans laquelle la séquence d'aminoacides de la SEQ ID N° 3 a été modifiée par délétion, substitution ou addition d'aminoacides de telle sorte que la polyhydroxyalcanoate-synthase résultante ait encore l'activité de conversion de l'acide 5(4-fluorophényl)valérique en un polymère comprenant de l'acide 3-hydroxy-5-(4-fluorophényl)-valérique comme motif monomère.

7. Gène de polyhydroxyalcanoate-synthase comprenant une séquence d'ADN codant pour la séquence d'aminoacides de la polyhydroxyalcanoate-synthase suivant la revendication 6.

8. Gène de polyhydroxyalcanoate-synthase comprenant une séquence d'ADN de la SEQ ID N° 4 codant pour la séquence d'aminoacides de la SEQ ID N° 3.

9. Vecteur recombinant comprenant le gène suivant la revendication 3, 4, 7 ou 8 comme gène de polyhydroxyalcanoate-synthase.

10. Microorganisme transformé, qui a été transformé par introduction du vecteur recombinant suivant la revendication 9.

11. Procédé pour la préparation d'un polyhydroxyalcanoate, comprenant les étapes consistant à cultiver le microorganisme transformé suivant la revendication 10 dans un milieu contenant un substrat pour une polyhydroxyalcanoate-synthase et à isoler le polyhydroxyalcanoate de la culture obtenue.

12. Procédé pour la production d'une polyhydroxyalcanoate-synthase, comprenant les étapes consistant à cultiver le microorganisme transformé suivant la revendication 10 et à amener le microorganisme transformé à produire la polyhydroxyalcanoate-synthase.


### SEQUENCE LISTING

<110> CANON INC.
<120> Polyhydroxyalkanoate Synthase and Gene Encoding the Same Enzyme
<130> 4051020
<160> 10
<170> Microsoft Word
<210> 1
   <211> 559
   <212> PRT
   <213> Pseudomonas cichorii H45 ; FERM BP-7374
<400> 1
   Met Ser Asn Lys Asn Asn Asp Asp Leu Lys Asn Gln Ala Ser Glu Asn
   1 5 10 15
   Thr Leu Gly Leu Asn Pro Val Val Gly Leu Arg Gly Lys Asp Leu Leu
   20 25 30
   Ala Ser Ala Arg Met Val Leu Arg Gln Ala Ile Lys Gln Pro Ile His
   35 40 45
   Ser Ala Arg His Val Ala His Phe Gly Leu Glu Leu Lys Asn Val Leu
   50 55 60
   Leu Gly Lys Ser Glu Leu Leu Pro Thr Ser Asp Asp Arg Arg Phe Ala
   65 70 75 80
   Asp Pro Ala Trp Ser Gln Asn Pro Leu Tyr Lys Arg Tyr Leu Gln Thr
   85 90 95
   Tyr Leu Ala Trp Arg Lys Glu Leu His Asp Trp Ile Gly Asp Ser Asn
   100 105 110
   Leu Pro Pro Lys Asp Val Ser Arg Gly His Phe Val Ile Asn Leu Met
   115 120 125
   Thr Glu Ala Phe Ala Pro Thr Asn Thr Ala Ala Asn Pro Ala Ala Val
   130 135 140
   Lys Arg Phe Phe Glu Thr Gly Gly Lys Ser Leu Leu Asp Gly Leu Ser
   145 150 155 160
   His Leu Ala Lys Asp Leu Val His Asn Gly Gly Met Pro Ser Gln Val
   165 170 175
   Asn Met Gly Ala Phe Glu Val Gly Lys Thr Leu Gly Val Thr Glu Gly
   180 185 190
   Ala Val Val Phe Arg Asn Asp Val Leu Glu Leu Ile Gln Tyr Lys Pro
   195 200 205
   Ile Thr Glu Gln Val His Glu Arg Pro Leu Leu Val Val Pro Pro Gln
   210 215 220
   Ile Asn Lys Phe Tyr Val Phe Asp Leu Ser Pro Glu Lys Ser Leu Ala
   225 230 235 240
   Arg Phe Cys Leu Arg Asn Asn Val Gln Thr Phe Ile Val Ser Trp Arg
   245 250 255
   Asn Pro Thr Lys Glu Gln Arg Glu Trp Gly Leu Ser Thr Tyr Ile Glu
   260 265 270
   Ala Leu Lys Glu Ala Val Asp Val Val Thr Ala Ile Thr Gly Ser Lys
   275 280 285
   Asp Val Asn Met Leu Gly Ala Cys Ser Gly Gly Ile Thr Cys Thr Ala
   290 295 300
   Leu Leu Gly His Tyr Ala Ala Ile Gly Glu Asn Lys Val Asn Ala Leu
   305 310 315 320
   Thr Leu Leu Val Ser Val Leu Asp Thr Thr Leu Asp Ser Asp Val Ala
   325 330 335
   Leu Phe Val Asp Glu Gln Thr Leu Glu Ala Ala Lys Arg Gln Ser Tyr
   340 345 350
   Gln Ala Gly Val Leu Glu Gly Arg Asp Met Ala Lys Val Phe Ala Trp
   355 360 365
   Met Arg Pro Asn Asp Leu Ile Trp Asn Tyr Trp Val Asn Asn Tyr Leu
   370 375 380
   Leu Gly Asn Glu Pro Pro Val Phe Asp Ile Leu Phe Trp Asn Asn Asp
   385 390 395 400
   Thr Thr Arg Leu Pro Ala Ala Phe His Gly Asp Leu Ile Glu Met Phe
   405 410 415
   Lys Ser Asn Pro Leu Thr Arg Ala Asp Ala Leu Glu Val Cys Gly Thr
   420 425 430
   Pro Ile Asp Leu Lys Lys Val Thr Ala Asp Ile Phe Ser Leu Ala Gly
   435 440 445
   Thr Ser Asp His Ile Thr Pro Trp Arg Ser Cys Tyr Lys Ser Ala Gln
   450 455 460
   Leu Phe Gly Gly Asn Val Glu Phe Val Leu Ser Ser Ser Gly His Ile
   465 470 475 480
   Gln Ser Ile Leu Asn Pro Pro Gly Asn Pro Lys Ser Arg Tyr Met Thr
   485 490 495
   Ser Thr Gln Met Pro Ala Asp Ala Asp Asp Trp Gln Glu Glu Ser Thr
   500 505 510
   Lys His Ala Asp Ser Trp Trp Leu His Trp Gln Ala Trp Gln Ala Gln
   515 520 525
   Arg Ser Gly Asn Leu Lys Lys Ala Pro Ala Lys Leu Gly Asn Lys Ala
   530 535 540
   Tyr Pro Ala Gly Glu Ala Ala Pro Gly Thr Tyr Val His Glu Arg
   545 550 555
<210> 2
   <211> 1680
   <212> DNA
   <213> Pseudomonas cichorii H45; FERM BP-7374
<400> 2
   atgagtaaca agaataacga tgacttgaag aatcaagcct cggaaaacac 50
   cttggggctg aatcctgtcg ttggactgcg tggaaaggat ctactggctt 100
   ctgctcgcat ggtactcagg caggccatca aacaaccgat tcacagcgcc 150
   aggcatgttg cgcatttcgg ccttgaactc aagaacgtgc tgctcggcaa 200
   atccgagctg ctaccgacca gcgatgaccg tcgcttcgcg gatccggcct 250
   ggagccagaa cccgctctac aaacgttatc tgcaaaccta cctggcgtgg 300
   cgcaaggaac tccacgactg gatcggcgac agcaacctgc cgcccaagga 350
   cgtcagccgc gggcatttcg tgatcaacct catgaccgaa gccttcgccc 400
   cgaccaacac ggcggccaac ccggcggcgg tcaagcgctt cttcgaaacc 450
   ggtggcaaaa gcctgctcga tggcctctcg catctggcca aggacctggt 500
   gcataacggc ggcatgccga gccaggtcaa catgggcgca ttcgaggtcg 550
   gcaagaccct tggtgtgacc gagggcgcag tggtctttcg caacgacgtg 600
   ctggagctga tccagtacaa gccgatcacc gagcaggtgc atgaacgccc 650
   actgctggtg gtaccgccgc agatcaacaa gttctacgtt ttcgacctga 700
   gcccggaaaa aagcctggcg cggttctgcc tgcgcaacaa cgtgcagacc 750
   ttcatcgtca gctggcgcaa cccgaccaag gagcagcgcg agtggggcct 800
   gtcgacctac atcgaagcgc tcaaggaagc ggttgatgtg gtcaccgcca 850
   ttaccggcag caaagacgtg aacatgctcg gtgcctgctc cggcggcatc 900
   acctgcaccg cgctgctggg ccactacgca gcaatcggcg agaacaaggt 950
   caacgccctg accctgctgg tcagcgtgct cgacaccacc ctggacagcg 1000
   acgtggccct gttcgtcgac gagcagaccc tcgaagccgc caagcgccaa 1050
   tcgtaccagg ccggcgtgct ggaaggccgc gacatggcca aggtcttcgc 1100
   ctggatgcgc cccaacgacc tgatctggaa ctactgggtc aacaactacc 1150
   tgttgggcaa cgaaccgccg gttttcgaca ttctgttctg gaacaacgac 1200
   accacccggt tgcctgcggc gttccatggc gacctgatcg aaatgttcaa 1250
   aagcaatcca ttgacccgcg ccgatgcact ggaagtgtgc ggcacgccga 1300
   tcgacctgaa gaaggttacc gccgacatct tctcgctggc cggcaccagc 1350
   gaccacatca cgccgtggcg ctcctgctac aagtcggcgc aactgttcgg 1400
   cggcaacgtt gaattcgtgc tgtccagcag cgggcacatc cagagcattc 1450
   tgaacccgcc gggcaatccg aaatcgcgct acatgaccag cacccaaatg 1500
   cccgccgatg ccgatgactg gcaggaagag tcgaccaagc acgccgactc 1550
   ctggtggctg cactggcagg catggcaggc acagcgttcg ggcaacctga 1600
   aaaaagcacc ggcaaaactg ggcaacaagg cctacccggc aggggaagcc 1650
   gcaccgggca cttacgtgca tgagcggtaa 1680
<210> 3
   <211> 560
   <212> PRT
   <213> Pseudomonas cichorii H45; FERM BP-7374
<400> 3
   Met Arg Glu Lys Pro Ala Arg Asp Ser Leu Pro Thr Pro Ala Ala Phe
   1 5 10 15
   Ile Asn Ala Gln Ser Ala Ile Thr Gly Leu Arg Gly Arg Asp Leu Leu
   20 25 30
   Ser Thr Leu Arg Ser Val Ala Ala His Gly Leu Arg Asn Pro Val His
   35 40 45
   Ser Ala Arg His Ala Leu Lys Leu Gly Gly Gln Leu Gly Arg Val Leu
   50 55 60
   Leu Gly Glu Thr Leu His Pro Thr Asn Pro Gln Asp Thr Arg Phe Ala
   65 70 75 80
   Asp Pro Ala Trp Ser Leu Asn Pro Phe Tyr Arg Arg Ser Leu Gln Ala
   85 90 95
   Tyr Leu Ser Trp Gln Lys Gln Val Lys Ser Trp Ile Asp Glu Ser Asp
   100 105 110
   Met Ser Pro Asp Asp Arg Ala Arg Ala His Phe Ala Phe Ala Leu Leu
   115 120 125
   Asn Asp Ala Val Ser Pro Ser Asn Thr Leu Leu Asn Pro Leu Ala Val
   130 135 140
   Lys Glu Leu Phe Asn Ser Gly Gly Asn Ser Leu Val Arg Gly Ile Gly
   145 150 155 160
   His Leu Val Asp Asp Leu Leu His Asn Asp Gly Leu Pro Arg Gln Val
   165 170 175
   Thr Lys Gln Ala Phe Glu Val Gly Lys Thr Val Ala Thr Thr Thr Gly
   180 185 190
   Ala Val Val Phe Arg Asn Glu Leu Leu Glu Leu Ile Gln Tyr Lys Pro
   195 200 205
   Met Ser Glu Lys Gln Tyr Ser Lys Pro Leu Leu Val Val Pro Pro Gln
   210 215 220
   Ile Asn Lys Tyr Tyr Ile Phe Asp Leu Ser Pro His Asn Ser Phe Val
   225 230 235 240
   Gln Tyr Ala Leu Lys Asn Gly Leu Gln Thr Phe Met Ile Ser Trp Arg
   245 250 255
   Asn Pro Asp Val Arg His Arg Glu Trp Gly Leu Ser Thr Tyr Val Glu
   260 265 270
   Ala Val Glu Glu Ala Met Asn Val Cys Arg Ala Ile Thr Gly Ala Arg
   275 280 285
   Glu Val Asn Leu Met Gly Ala Cys Ala Gly Gly Leu Thr Ile Ala Ala
   290 295 300
   Leu Gln Gly His Leu Gln Ala Lys Arg Gln Leu Arg Arg Val Ser Ser
   305 310 315 320
   Ala Thr Tyr Leu Val Ser Leu Leu Asp Ser Gln Leu Asp Ser Pro Ala
   325 330 335
   Ser Leu Phe Ala Asp Glu Gln Thr Leu Glu Ala Ala Lys Arg Arg Ser
   340 345 350
   Tyr Gln Lys Gly Val Leu Asp Gly Arg Asp Met Ala Lys Val Phe Ala
   355 360 365
   Trp Met Arg Pro Asn Asp Leu Ile Trp Ser Tyr Phe Val Asn Asn Tyr
   370 375 380
   Leu Leu Gly Lys Glu Pro Pro Ala Phe Asp Ile Leu Tyr Trp Asn Asn
   385 390 395 400
   Asp Ser Thr Arg Leu Pro Ala Ala Leu His Gly Asp Leu Leu Asp Phe
   405 410 415
   Phe Lys His Asn Pro Leu Thr His Pro Gly Gly Leu Glu Val Cys Gly
   420 425 430
   Thr Pro Ile Asp Leu Gln Lys Val Thr Val Asp Ser Phe Ser Val Ala
   435 440 445
   Gly Ile Asn Asp His Ile Thr Pro Trp Asp Ala Val Tyr Arg Ser Thr
   450 455 460
   Leu Leu Leu Gly Gly Glu Arg Arg Phe Val Leu Ser Asn Ser Gly His
   465 470 475 480
   Val Gln Ser Ile Leu Asn Pro Pro Ser Asn Pro Lys Ala Asn Tyr Val
   485 490 495
   Glu Asn Gly Lys Leu Ser Ser Asp Pro Arg Ala Trp Tyr Tyr Asp Gly
   500 505 510
   Arg His Val Asp Gly Ser Trp Trp Thr Gln Trp Leu Ser Trp Val Gln
   515 520 525
   Glu Arg Ser Gly Ala Gln Lys Glu Thr His Met Ala Leu Gly Asn Gln
   530 535 540
   Asn Tyr Pro Pro Met Glu Ala Ala Pro Gly Thr Tyr Val Arg Val Arg
   545 550 555 560
<210> 4
   <211> 1683
   <212> DNA
   <213> Pseudomonas cichorii H45; FERM BP-7374
<400> 4
   atgcgcgaga aaccagcgag ggattcgtta ccgactcccg ccgcgttcat 50
   caatgcacag agtgcgatta ccggcctgcg cggtcgggat ctgttatcga 100
   ccctgcgcag tgtggccgcc catggcttgc gcaatccggt gcacagtgcc 150
   cgacatgccc tcaaactcgg cggccagctt ggtcgtgtgc tgctgggtga 200
   aaccctgcac ccgaccaacc cgcaggacac tcgcttcgcc gatccggcgt 250
   ggagcctcaa cccgttttac cggcgcagcc tgcaggccta tctgagctgg 300
   cagaagcagg tcaaaagctg gatcgacgag agcgacatga gcccggacga 350
   ccgcgcccgc gcccacttcg ctttcgcctt gctcaacgac gccgtatcgc 400
   cctccaacac cctgctcaat ccattggcgg tcaaggagct cttcaattcc 450
   ggtggtaaca gcctggtgcg tggcatcggc catctggtgg acgatctgct 500
   gcacaacgat ggcttgcccc ggcaagtcac caagcaagcg ttcgaggtcg 550
   gcaagacggt cgccaccacc accggtgccg tggtgtttcg caacgaactg 600
   ctggagttga tccagtacaa gccgatgagc gaaaagcagt attccaagcc 650
   cctgctggtg gtgccgccac aaatcaacaa gtactacatt ttcgacctga 700
   gcccccacaa cagcttcgtc cagtacgcgc tgaaaaacgg cctgcagacc 750
   ttcatgatca gctggcgcaa cccggatgtg cgtcaccgtg aatgggggct 800
   ctcgacctac gtggaagccg tggaagaggc catgaatgtg tgccgggcga 850
   tcaccggcgc acgcgaggtc aacctgatgg gcgcctgcgc cggcgggttg 900
   accattgccg cgttgcaagg ccacttgcag gccaagcggc aactgcgcag 950
   ggtgtccagt gcgacttatc tggtgagcct gctcgacagc cagctggata 1000
   gccccgcttc gctgttcgcc gacgagcaga ctctggaggc cgccaagcgc 1050
   cgctcctatc agaaaggtgt gctggacggc cgcgacatgg ccaaggtctt 1100
   cgcctggatg cgccccaacg atttgatctg gagctacttc gtcaacaact 1150
   acctgttggg caaggagccg ccggcgttcg acatcctcta ctggaacaac 1200
   gacagcacgc gcctgccggc cgccctgcat ggcgacctgc tggacttctt 1250
   caagcacaac ccgctgaccc acccgggcgg cctggaagtg tgtggcacgc 1300
   cgatcgattt gcagaaggtc accgtcgaca gcttcagcgt cgccggcatc 1350
   aacgatcaca tcacgccgtg ggatgcggtg tatcgctcga cgctgttgct 1400
   cggtggcgag cggcgctttg tgctatccaa cagcggtcat gtgcagagca 1450
   tcctcaaccc gccgagcaac ccgaaagcca actacgtcga aaacggcaaa 1500
   ctgagcagcg acccccgcgc ctggtactac gacggcaggc atgtcgacgg 1550
   cagttggtgg acccaatggc tgagctgggt tcaggaacgc tccggcgcac 1600
   agaaggaaac ccacatggcg ctcggcaacc agaactatcc accgatggaa 1650
   gctgcgcccg gtacctacgt acgtgtgcgc tga 1683
<210> 5
   <211> 20
   <212> DNA
   <213> Artificial Sequence
<220>
   <223> Primer for PCR multiplication
<400> 5
   tgctggaact gatccagtac 20
<210> 6
   <211> 23
   <212> DNA
   <213> Artificial Sequence
<220>
   <223> Primer for PCR multiplication
<400> 6
   gggttgagga tgctctggat gtg 23
<210> 7
   <211> 30
   <212> DNA
   <213> Artificial Sequence
<220>
   <223> Primer for PCR multiplication
<400> 7
   ctacaaagct tgacccggta ctcgtctcag 30
<210> 8
   <211> 28
   <212> DNA
   <213> Artificial Sequence
<220>
   <223> Primer for PCR multiplication
<400> 8
   cgagcaagct tgctcctaca gtagggcg 28
<210> 9
   <211> 29
   <212> DNA
   <213> Artificial Sequence
<220>
   <223> Primer for PCR multiplication
<400> 9
   gttttaagct tgaagacgaa ggagtgttg 29
<210> 10
   <211> 29
   <212> DNA
   <213> Artificial Sequence
   <220>
   <223> Primer for PCR multiplication
<400> 10
   atcgcaagct tactcgctcc taccgggtc 29